# EUROPEAN PATENT APPLICATION

(11) **EP 4 253 391 A1**
(43) Date of publication of application: **04.10.2023**
(21) Application number: 21897040.8
(22) Date of filing: 24.11.2021
(51) Int. Cl.: C07F 15/00, A61P 35/00

(54) **RADIATION-ACTIVATED TETRAVALENT PLATINUM COMPLEX AND USE THEREOF**

(30) Priority: 25.11.2020 CN 202011337782
(71) Applicant: Peking University, Beijing 100871 (CN)
(72) Inventor: LIU, Zhibo, Beijing 100871 (CN); GUO, Zijian, Beijing 100871 (CN); FU, Qunfeng, Beijing 100871 (CN)
(74) Representative: Cabinet Becker et Associés
(86) International application number: PCT/CN2021/132884
(87) International publication number: WO 2022/111540

(57) **Abstract**

The present disclosure provides a complex of general formula (I), wherein M is tetravalent platinum; L is independently a neutral ligand or an anionic ligand when appearing each time; x is an integer of 1-5; P is a precursor ligand, the precursor ligand being a ligand of a tetravalent platinum ion which can be released from the complex after irradiation and converted into a functional molecule D to achieve functions such as medicine, fluorescence detection, or a functional material.

Lₓ-M-P (I)

## Description

### FIELD OF THE INVENTION

The present disclosure relates to the field of radiation chemistry, in particular to a radiation-activated tetravalent platinum complex and a use thereof.

### BACKGROUND OF THE INVENTION

Cancer is the second leading killer threatening people's health. At present, chemotherapy is the main clinical treatment for cancer. Platinum-based drugs have high-efficiency and broad-spectrum anti-cancer activity, thereby having become clinically important first-line chemotherapy drugs, and being widely used in the treatment of common malignant tumors such as lung cancer, bladder cancer, ovarian cancer, cervical cancer, esophageal cancer, gastric cancer, colorectal cancer, and head and neck tumors. The first generation of platinum-based anticancer drugs is represented by cisplatin; the second generation of platinum-based anticancer drugs is represented by carboplatin, nedaplatin, and cycloplatin; and the third generation of platinum-based anticancer drugs is represented by oxaliplatin and lobaplatin. These platinum-based anticancer drugs are mainly complexes of divalent platinum. Tetravalent platinum compounds themselves have low killing ability to cancer cells, and can exert anticancer activity by being reduced and releasing divalent platinum under physiological conditions. Tetravalent platinum compounds not only retain the broad-spectrum and high-efficiency anticancer advantages of traditional divalent platinum drugs, but also bring other unique advantages because of the coordination structure of tetravalent platinum different from divalent platinum. Tetravalent platinum has a d²sp³ hexa-coordination structure, and has stability stronger than that of divalent platinum, thereby having higher blood stability. The tetravalent platinum complex possesses two additional ligands in the axial direction, which provides more options for the design of platinum-based drugs. Tetravalent platinum can not only be subjected to structural modification on the horizontal ligand but also on the axial ligand, such as introducing functional groups for lipid-water adjusting, enzyme targeting, DNA targeting, or serum albumin targeting into the axial ligand.

At present, many studies have been carried out on platinum-based compounds, and the defects of platinum-based drugs, such as strong toxic side effects, low absorption, poor targeting, and serious drug resistance, have been discovered. Therefore, there is still a need to develop a novel platinum-based compound. However, at present, there is no report on the introduction of a radiation-responsive molecule into a ligand of a tetravalent platinum-based compound.

In addition, there is no report on the use of radiation to release functional molecules from ligands of tetravalent platinum-based compounds to achieve functions such as medicine, fluorescence detection and functional materials.

### SUMMARY OF THE INVENTION

One aspect of the present disclosure provides a metal complex of general formula (I),

Lₓ-M-P (I)

wherein M is tetravalent platinum; L is independently for each occurrence a neutral ligand or an anionic ligand; x is an integer from 1 to 5; and P is a precursor ligand, the precursor ligand being a ligand of the tetravalent platinum ion which can be released from the complex after irradiation and converted into a functional molecule D.

In some embodiments, the functional molecule D is selected from drug molecules, fluorescent molecules, and functional material molecules. In some preferred embodiments, the functional molecule D is an anticancer drug molecule. In some more preferred embodiments, the functional molecule D is an anticancer drug molecule, and at least one L has a group targeting tumor cells. For example, the L having a group targeting tumor cells includes a sugar transporter targeting group, a glutamine receptor targeting group, a phosphate receptor targeting group, an epidermal growth factor receptor targeting group, an integrin targeting group, an energy metabolism enzyme targeting group, a chondriosome targeting group, a serum albumin targeting group, an inflammatory factor targeting group, a DNA targeting group, a histone deacetylase (HDAC) targeting group, a P53 gene activator group, a tubulin inhibitor group, a cyclin-dependent kinase inhibitor group, or an indoleamine 2,3-dioxygenase inhibitor group.

In some embodiments, the functional molecule D is selected from monomethyl auristatin E, monomethyl auristatin F, ibrutinib, acatinib, zanubrutinib, doxorubicin, mitomycin-C, mitomycin-A, daunorubicin, aminopterin, actinomycin, bleomycin, 9-aminocamptothecin, N8-acetylspermidine, 1,2-bis(methylsulfonyl)-1-(2-chloroethyl)hydrazine, yunnanmycin, gemcitabine, cytarabine, dolastatin, dacarbazine, 5-fluorouracil, paclitaxel, docetaxel, gemcitabine, cytarabine; and 6-mercaptopurine. In some preferred embodiments, the functional molecule D is monomethyl auristatin E, monomethyl auristatin F, or 5-fluorouracil.

In some embodiments, at least one ligand L is selected from: NH₃, ethylenediamine, F⁻, Cl⁻, oxalate, malonate, 1,2-diaminocyclohexane, 1,2-diaminobenzene, 2-aminopropane, aminocyclohexane, cyclobutane-1,1-dicarboxylate, glycolate, lactate, aminocyclohexane, 2-isopropyl-4,5-bis(aminomethyl)-1,3-dioxolane, 5-(triphenylphosphonio)valerate, succinate, 6-(2,5-dioxo-2,5-dihydro-1H-pyrrol-1-yl)hexanoate, porphyrin, acetate, and propionate.

In some embodiments, one ligand L is an axial ligand capable of being released from the complex after irradiation and converted into a functional molecule which may be the same as or different from the functional molecule D.

In some embodiments, P is an axial ligand.

In some embodiments, P is ⁻O-C(=O)-X-Y, wherein
X is -NH-, -NR-, -O-, or -S-, R is an optionally substituted C₁₋₁₀ alkyl, and HXY constitutes the functional molecule D; or
X is -CH₂-, -CRH-, or -CR₂-, wherein R is independently for each occurrence an optionally substituted C₁₋₁₀ alkyl, or two R groups are taken together with the carbon atom to which they are attached to form a 5-membered ring or a 6-membered ring, and HOOCXY constitutes the functional molecule D.

Another aspect of the present disclosure also provides a use of the above metal complex of the general formula (I) in the fields of medicine, detection or functional materials, etc., wherein the metal complex of general formula (I) releases a functional molecule from a ligand after irradiation to realize functions such as medicine, fluorescence detection or functional materials.

### BRIEF DESCRIPTION OF THE DRAWINGS

In order to illustrate the technical solutions of the examples of the present disclosure more clearly, the drawings of the examples will be briefly introduced below. Apparently, the drawings in the following description only relate to some examples of the present disclosure, rather than limiting the present disclosure.
Fig. 1 shows the ligand release percentage of 10 µM tetravalent platinum complexes 19-31 after 60 Gy of X-ray irradiation.
Fig. 2 shows the reaction schematic diagram, the fluorescence change diagram, and the UPLC change diagram for screening tetravalent platinum compounds 32, 33, and 34 that are stable in vivo.
FIG. 3 shows the result of in vivo activation test of tetravalent platinum compound 35 with oxaliplatin as a parent.
Fig. 4 shows the result of activation test of a radiation-responsive antibody-drug conjugate with platinum as a linker.
Fig. 5 shows the broad-spectrum and high-efficiency release of FDA-approved Pt(II) drugs from Pt(IV) complexes driven by radiation.
Fig. 6 shows the construction and characterization of ADCs whose drug release can be driven by radiotherapy.
Fig. 7 shows the pharmacokinetic properties of OxaliPt(IV)-ADC.

### DETAILED DESCRIPTION OF THE INVENTION

In order to make the purpose, technical solutions and advantages of the examples of the present disclosure clearer, the technical solutions of the examples of the present disclosure will be clearly and completely described below in conjunction with the drawings of the examples of the present disclosure. Apparently, the described examples are a part of the examples of the present disclosure, not all of the examples of the present disclosure. Based on the described examples of the present disclosure, all other examples obtained by those of ordinary skill in the art without creative effort shall fall within the protection scope of the present invention.

The present disclosure may be embodied in other specific forms without departing from essential attributes of the present disclosure. It should be understood that any and all embodiments of the present disclosure can be combined with technical features in another embodiment or other embodiments to obtain additional embodiments under the premise of no conflict. The additional embodiments resulting from such combinations are included by the present disclosure.

All publications and patents mentioned in this disclosure are hereby incorporated by reference into this disclosure in their entirety. To the extent that usage or terminology used in any publications and patents incorporated by reference conflicts with usage or terminology used in the present disclosure, the usage and terminology in the present disclosure shall control.

The section headings used herein are for the purpose of organizing the article only and should not be construed as limitations on the subject matter described.

Unless defined otherwise, all technical and scientific terms used herein have their ordinary meanings in the art to which the claimed subject matter belongs. In the event that more than one definition exists for a term, the definition herein controls.

Unless otherwise stated, when any type of range is disclosed or claimed (e.g., number of ligands), it is intended that each possible value that the range could reasonably encompass is individually disclosed or claimed, including any sub-ranges encompassed therein. For example, the numerical range of ligand L herein, such as 1-5, indicates an integer within the range, wherein 1-5 should be understood to include 1, 2, 3, 4, 5, and also include the ranges of 1-4 and 1-3.

The description of the present disclosure should be construed in accordance with the laws and principles of chemical bonding. In some cases it may be possible to remove a hydrogen atom in order to accommodate a substituent at a given position.

The words "comprising", "including" or "containing" and similar words used in the present disclosure mean that an element appearing before the words covers elements listed after the words and equivalents thereof, and does not exclude any unrecited element. The term "comprising" or "including (containing)" used herein can be open, semi-closed and closed. In other words, the term also includes "consisting essentially of" or "consisting of".

As used herein, the terms "moiety", "structural moiety", "chemical moiety", "group", and "chemical group" refer to a specific segment or functional group in a molecule. A chemical moiety is generally considered to be a chemical entity embedded in or attached to a molecule.

It should be understood that a singular form (e.g., "a") as used in this disclosure may include plural referents unless otherwise specified.

Unless otherwise indicated, this disclosure employs standard nomenclature and standard laboratory procedures and techniques of analytical chemistry, synthetic organic chemistry, and coordination chemistry. Unless otherwise stated, the present disclosure adopts traditional methods of mass spectrometry and elemental analysis, and the steps and conditions can refer to the conventional operation steps and conditions in the art.

The reagents and starting materials used in the present disclosure are commercially available or can be prepared by conventional chemical synthesis methods.

The term "optional" used herein to describe a situation means that the situation may or may not occur. For example, being optionally fused to a ring means that it is fused to or not fused to a ring. For example, the term "optionally substituted" as used herein refers to being unsubstituted or having at least one non-hydrogen substituent that does not destroy the desired property possessed by the unsubstituted analog.

In the present disclosure, unless otherwise specified, the number of "substitution" can be one or more. When the number of "substitution" is more than one, it can be 2, 3 or 4. Moreover, when the number of the "substitution" is more than one, the "substitution" may be the same or different.

In the present disclosure, the position of "substitution" can be arbitrary unless otherwise specified.

The term "axial ligands" as used herein refers to the two axial ligands in the d²sp³ hexa-coordinated structure of tetravalent platinum, which are released from a complex after the complex is reduced by irradiation.

The term "lateral ligands" as used herein refers to the four lateral ligands in the d²sp³ hexa-coordinated structure of tetravalent platinum, which can still be coordinated with a divalent platinum ion or can be released from a complex after the complex is reduced by irradiation.

As used herein, the term "neutral ligand" or "anionic ligand" refers to a ligand capable of being coordinated with platinum, wherein the ligand is uncharged or negatively charged as a whole, but may locally have a cation such as triphenylphosphonium or ammonium.

As used herein, the term "C₁-C₁₀ alkyl" refers to a straight or branched alkane chain containing 1 to 10 carbon atoms. For example, representative examples of C₁-C₆ alkyl include, but are not limited to, methyl (C₁), ethyl (C₂), n-propyl (C₃), isopropyl (C₃), n-butyl (C₄), tert-butyl (C₄), sec-butyl (C4), isobutyl (C₄), n-pentyl (C₅), 3-pentyl (C₅), neopentyl (C₅), 3-methyl-2-butanyl (C₅), tert-amyl (C₅), and n-hexyl (C₆), etc. The term "lower alkyl" refers to straight or branched chain alkyl having 1 to 4 carbon atoms. "Substituted alkyl" refers to an alkyl group substituted with one or more substituents, preferably 1 to 4 substituents, at any available point of attachment. The term "haloalkyl" refers to an alkyl group having one or more halogen substituents, including but is not limited to groups such as -CH₂Br, -CH₂I, -CH₂Cl, -CH₂F, -CHF₂, -CF₃, and the like.

The term "alkylene" as used herein refers to a divalent hydrocarbon group as described above for "alkyl" but having two points of attachment. For example, methylene is a -CH₂- group, and ethylene is a -CH₂-CH₂- group.

The terms "alkoxy" and "alkylthio" as used herein refer to an alkyl group as described above linked via an oxygen bond (-O-) or a sulfur bond (-S-), respectively. The terms "substituted alkoxy" and "substituted alkylthio" refer to a substituted alkyl group linked via an oxygen or sulfur bond, respectively. "Lower alkoxy" is the group OR, wherein R is lower alkyl (alkyl containing 1 to 4 carbon atoms).

The term "halogen" as used herein refers to fluorine, chlorine, iodine or bromine.

The radiation sources of the present disclosure may be alpha, beta, gamma rays produced by the decay of radionuclides. X-rays, gamma rays, energetic electrons, protons, heavy ions produced by external radiation sources, alpha particles produced by boron neutron capture therapy (BNCT), and other possible external or internal sources of radiation are also applicable to the present disclosure.

The high-energy rays used in radiotherapy have high spatial and temporal resolution, and high tissue penetration ability, and at the same time are highly clinically relevant. Using high-energy rays in radiotherapy to activate precursor molecules and carry out chemical reactions in vivo has both basic research value and clinical application value.

The chemical reaction activated by high-energy rays involves the radiolysis of water by the rays to produce a large number of reactive species. The reactive species are then reacted with a target substrate. In the products of water radiolysis, the compounds with the highest yields are hydroxyl radicals and hydrated electrons.

A living body is generally a reducing environment, and a large number of substances such as glutathione and vitamin C can quench hydroxyl radicals and increase the production of hydrated electrons. In this condition, using hydrated electrons to carry out chemical reactions will be a big breakthrough in chemistry in vivo.

High-energy rays (such as X-rays and γ-rays) can be used as external stimuli to chemically react a complex having precursor ligands to release functional molecules. Due to the high penetrating power of the rays, as well as the high spatiotemporal resolution of the rays, the precursor ligands can be activated very efficiently by radiotherapy apparatus. For example, X-ray irradiation as an external trigger for activating precursor ligands can control radiation-induced chemical reactions in space and time, and thus can precisely control the area, time and dose of such precursor ligands converted to their active forms.

The radiochemical change of molecules is the material basis for the study of all radiochemical effects. There are two main types of radiochemical effects: direct effects in which ionizing radiation causes direct chemical changes in target molecules, and indirect effects in which radiation deposits on environmental molecules and then causes indirect chemical interactions in target molecules. Direct and indirect effects exist simultaneously, but indirect effect dominates in vivo. Because the tissue comprises 70-80% water, various active substances are mainly produced through the radiolysis of water (**Scheme 1a**), wherein substances with the highest yields are hydroxyl radicals (OH) and hydrated electrons (e_{aq}⁻). The radiolysis of water is completed within 10⁻⁴ seconds, so the radiation-induced reaction tends to occur instantaneously, and thus has strong controllability. OH-induced cleavage chemistry reaction and related fluorescent probes have been successfully used in bioimaging, but the rapid quenching of OH by the reducing tumor microenvironment hinders its development in living systems. However, the yield of radiation-induced e_{aq}⁻, another major product of water radiolysis, increases in reducing environments. Therefore, here we explore the feasibility of locally generating e_{aq}⁻ by precise radiotherapy to mediate chemical cleavage reactions (**Scheme 1b**), and the use of radiation as a chemical tool to release target molecules in a highly tumor-selective manner **(Scheme 1c).**

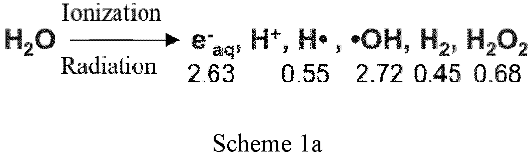

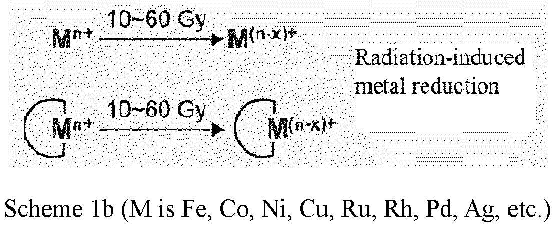

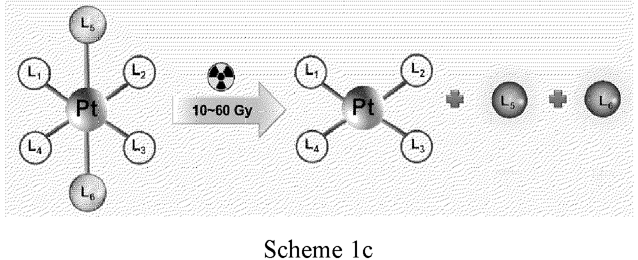

**Scheme 1. Radiation-induced controlled release of metal complexes in tumors. a,** Radiolysis of water by ionizing radiation. The G value of hydrated electrons is 2.63 (G value refers to the number of molecules formed by absorbing 100 eV energy in the system). **b**, Radiation-generated hydrated electrons can reduce metal ions and metal complexes. **c**, Pt(IV) complexes can be reduced by irradiation and release Pt(II) anticancer drugs and active axial ligands (such as fluorescent probes or anticancer drugs).

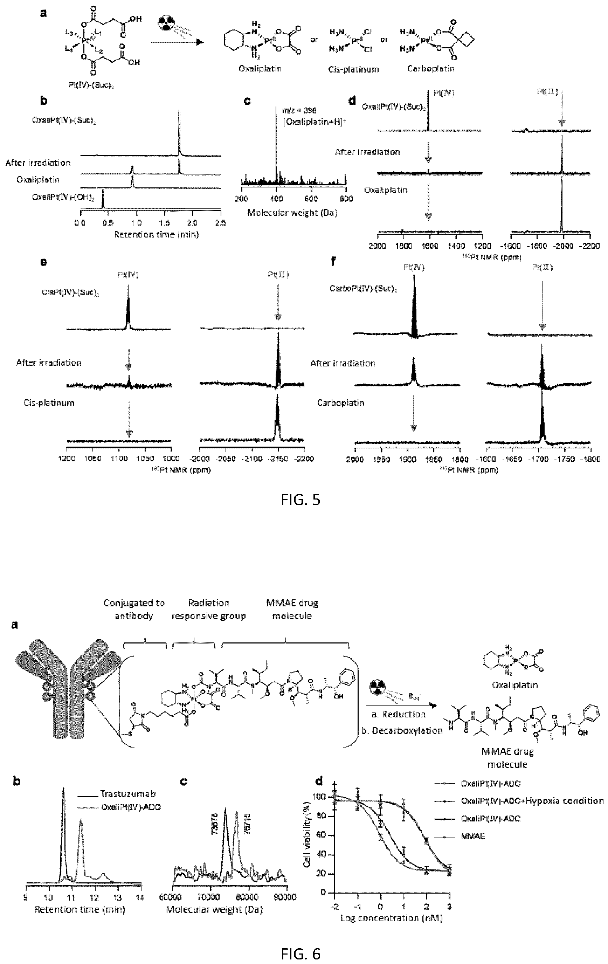

In order to explore the reaction process, the solution of oxaliPt(IV)-(Suc)₂ (80 mM, D₂O) was deoxygenated and then subjected to irradiation with 40 kGy of γ-rays (⁶⁰Co source, 200 Gy/min, 200 min, Fig. **5a****).** The irradiated solution was detected by UPLC-MS, and only one new peak was observed, wherein the retention time **(****Fig. 5b****)** and mass spectrometry signal (Fig. **5c****)** were both consistent with those of the oxaliplatin standard sample. The product was analyzed by nuclear magnetic resonance (NMR). ¹⁹⁵Pt-NMR showed that the peak of the Pt(IV) complex at 1615 ppm almost disappeared after irradiation (Fig. **5d** **top),** and a new singlet peak appeared at -1988 ppm (Fig. **5d** **middle**), which was within the chemical shift range of the Pt(II) complex and coincided with that of oxaliplatin (Fig. **5d** **bottom).** The above tests all proved that the release of axial ligands was caused by radiation reduction of Pt(IV) rather than hydrolysis.

To explore the generalizability of this strategy, we further conducted the same study on two other platinum-based drugs commonly used globally, carboplatin and cisplatin. NMR characterization revealed that cisPt(IV)-(Suc)₂ and carboPt(IV)-(Suc)₂ would release the corresponding Pt(II) drugs after γ-ray irradiation in D₂O **(****Fig. 5e****, f).** In view of the wide application of platinum-based drugs in chemotherapy, the strategy proposed in this work to control the release of Pt(II) by reduction of Pt(IV) prodrugs driven by radiation is very promising in realizing precision chemotherapy driven by radiotherapy.

Fig. 5 exemplifies the broad-spectrum and high-efficiency release of FDA-approved Pt(II) drugs from Pt(IV) complexes driven by radiation. **a**, Schematic illustration of release of Pt(II) drugs from Pt(IV) complexes driven by radiation. **b**, UPLC chromatograms of oxaliPt(IV)-(Suc)₂, oxaliPt(IV)-(Suc)₂ + radiation, oxaliplatin, and oxaliPt(IV)-(OH)₂, where oxaliplatin and oxaliPt(IV)-(OH)₂ were used as a reference. The main product released from oxaliPt(IV)-(Suc)₂ driven by radiation had the same retention time as that of oxaliplatin. The detector wavelength was set at 254 nm. **c**, MS of the product released from oxaliPt(IV)-(Suc)₂ driven by radiation showed that the released product was oxaliplatin. **d-f**, Study of Pt(II) drugs released from Pt(IV) complexes by nuclear magnetic resonance (NMR). **d**, ¹⁹⁵Pt-NMR spectra of oxaliPt(IV)-(Suc)₂ (1615 ppm, top), radiation product (-1988 ppm, middle) and external standard (bottom). **e**, ¹⁹⁵Pt-NMR spectra of cisPt(IV)-(Suc)₂ (1082 ppm, top), radiation product (-2150 ppm, middle) and external standard (bottom), **f,** ¹⁹⁵Pt-NMR spectra of carboPt(IV)-(Suc)₂ (1883 ppm, top), irradiation product (1707 ppm, middle) and external standard (bottom). ¹⁹⁵Pt-NMR spectrum showed that the release of FDA-approved Pt(II) drugs driven by radiation is effective and generally applicable to Pt(IV) complexes.

The present disclosure provides a metal complex of general formula (I),

Lₓ-M-P (I)

wherein M is tetravalent platinum; L is independently for each occurrence a neutral ligand or anionic ligand; x is an integer from 1 to 5; and P is a precursor ligand, the precursor ligand being a ligand of a tetravalent platinum ion which can be released from the complex and converted into a functional molecule D after irradiation.

L can be an axial ligand and/or a lateral ligand in the tetravalent platinum complex. Ligand L is a ligand capable of being coordinated with tetravalent platinum or divalent platinum. In a preferred embodiment, the ligand L is a ligand capable of being coordinated with tetravalent platinum or divalent platinum to form a stable complex. In a more preferred embodiment, the ligand L, which is a lateral ligand, still keeps stable complexes with platinum ions after irradiation.

The choice of x is such that all L and P meet the requirement of a total of six coordinations. When x is 2-5, those L in the complex can be the same or different. In some embodiments, both L and P are ligands having one coordination, and x is 5. In some embodiments, L is a ligand having one coordination, P is a ligand having two coordinations, and x is 4.

In some embodiments, the functional molecule D includes but is not limited to drug molecules, fluorescent molecules, or functional material molecules. The tetravalent platinum ion complex of the present disclosure is unique in that at least one axial ligand is released from the complex to form a functional molecule after irradiation. In one embodiment, both axial ligands are released from the complex after irradiation.

In some embodiments, the functional molecule D is an anticancer drug molecule. In some preferred embodiments, the functional molecule D is an anticancer drug molecule, and at least one L has a group targeting tumor cells. For example, the L having a group targeting tumor cells includes a sugar transporter targeting group, a glutamine receptor targeting group, a phosphate receptor targeting group, an epidermal growth factor receptor targeting group, an integrin targeting group, an energy metabolism enzyme targeting group, a chondriosome targeting group, a serum albumin targeting group, an inflammatory factor targeting group, a DNA targeting group, a histone deacetylase (HDAC) targeting group, a P53 gene activator group, a tubulin inhibitor group, a cyclin-dependent kinase inhibitor group, or an indoleamine 2,3-dioxygenase inhibitor group.

In some embodiments, the functional molecule D is selected from monomethyl auristatin E, monomethyl auristatin F, ibrutinib, acatinib, zanubrutinib, doxorubicin, mitomycin-C, mitomycin-A, daunorubicin, aminopterin, actinomycin, bleomycin, 9-aminocamptothecin, N8-acetylspermidine, 1,2-bis(methylsulfonyl)-1-(2-chloroethyl)hydrazine, yunnanmycin, gemcitabine, cytarabine, dolastatin, dacarbazine, 5-fluorouracil, paclitaxel, docetaxel, gemcitabine, cytarabine, and 6-mercaptopurine. In some preferred embodiments, the functional molecule D is monomethyl auristatin E, monomethyl auristatin F, or 5-fluorouracil.

In some embodiments, at least one ligand L is selected from the group consisting of: NH₃, ethylenediamine, F⁻, Cl⁻, oxalate, malonate, 1,2-diaminocyclohexane, 1,2-diaminobenzene, 2-aminopropane, aminocyclohexane, cyclobutane-1,1-dicarboxylate, glycolate, lactate, aminocyclohexane, 2-isopropyl-4,5-bis(aminomethyl)-1,3-dioxolane, 5-(triphenylphosphonio)valerate, succinate, 6-(2,5-dioxo-2,5-dihydro-1H-pyrrol-1-yl)hexanoate, porphyrin, acetate, and propionate.

In some embodiments, one ligand L is an axial ligand capable of being released from the complex after irradiation and converted into a functional molecule which may be the same as or different from the functional molecule D.

In some embodiments, P is an axial ligand.

In some embodiments, P is ⁻O-C(=O)-X-Y, wherein
X is -NH-, -NR-, -O-, or -S-, R is an optionally substituted C₁₋₁₀ alkyl group, and HXY constitutes the functional molecule D; or
X is -CH₂-, -CRH-, or -CR₂-, wherein R is independently for each occurrence an optionally substituted C₁₋₁₀ alkyl group, or two R groups are taken together with the carbon atom to which they are attached to form a 5-membered ring or a 6-membered ring, and HOOCXY constitutes the functional molecule D.

In one embodiment, the optionally substituted C₁₋₁₀ alkyl is C₁₋₁₀ alkyl or C₁₋₁₀ alkyl substituted by one or more substituents including, but not limited to, halogen, -R₁, -NR₁R₂, -CN, -NO₂, -N₃, -ORi, -SRi, -NHCOR₁, -O-COR₁, -CH=CR₁R₂, -C(=O)-R₁, -C(=O)-ORi, -C(=O)-Cl, -C(=O)-NH₂, -C(=O)-NH-R₁ and -C(=O)-NR₁R₂, wherein R₁ and R₂ are independently selected from H, C₁-C₆ alkyl, C₁-C₆ haloalkyl, C₂-C₆ alkenyl, C₃-C₁₀ cycloalkyl, C₆-C₂₀ aryl or heteroaryl with 5-20 ring atoms, wherein the alkyl, alkenyl, cycloalkyl, aryl and heteroaryl described for the substituent are optionally substituted by one or more of halogen, hydroxyl, mercapto, -NH₂, -CN, -NO₂, -N₃, -NHCOH, -OC(=O)H, -C(=O)H, -C(=O)-OH, -C(=O)-Cl, -C(=O)-NH₂, -C(=O)-NH-CH₃, -C(=O)-CH₃, -C(=O)-OCH₃, C₁-C₆ alkyl, C₁-C₆ alkoxy, C₁-C₆ haloalkyl, C₂-C₆ alkenyl, C₃-C₁₀ cycloalkyl, C₆-C₂₀ aryl, or heteroaryl with 5-20 ring atoms.

Another aspect of the present disclosure also provides a use of the above metal complex of general formula (I) in the fields of medicine, detection or functional materials, etc., wherein the metal complex of general formula (I) releases a functional molecule from a ligand after irradiation to realize functions such as medicine, fluorescence detection, or functional materials.

In some embodiments, on the one hand, the tetravalent platinum complex of the present disclosure is used as a precursor of a divalent platinum drug, and is reduced by hydrated electrons generated by irradiation to obtain a divalent platinum product having pharmaceutical activity such as anticancer activity; on the other hand, one or two axial ligand molecules in the tetravalent platinum complex undergo a chemical reaction by irradiation to release one or two functional molecules, which synergistically exert drug activity with the divalent platinum product, thereby reducing the effective dosage of the tetravalent platinum complex, and thus reducing the toxic and side effects of the drug.

In some embodiments, the tetravalent platinum complex of the present disclosure can introduce a targeting group into axial ligands or lateral ligands, thereby improving the targeting of the platinum compound. For example, groups such as a sugar transporter targeting group, a glutamine receptor targeting group, a phosphate receptor targeting group, an epidermal growth factor receptor targeting group, an integrin targeting group, an energy metabolism enzyme targeting group, a chondriosome targeting group, a serum albumin targeting group, an inflammatory factor targeting group, a DNA targeting group, a histone deacetylase (HDAC) targeting group, a P53 gene activator group, a tubulin inhibitor group, a cyclin-dependent kinase inhibitor group, or an indoleamine 2,3-dioxygenase inhibitor group can be introduced into axial ligands or lateral ligands of the tetravalent platinum complex to improve targeting. For details, please refer to "Chemistry Progress", 2018 30(6), pages 831-846, the entire content of which is incorporated herein as a part of the present disclosure.

In one embodiment, an antibody molecule or polypeptide is introduced into the lateral or axial ligands of tetravalent platinum. For the introduction of antibody molecules or polypeptides into ligands, please refer to, for example, Green Chem., 2020, 22, 2203-2212.

In one embodiment, Herceptin is incorporated into the axial or lateral ligands of the tetravalent platinum complex. In a preferred embodiment, Herceptin is incorporated into the axial ligand of the tetravalent platinum complex.

In one embodiment, a targeting ligand may comprise a maleimide group, wherein the maleimide group is attached to an antibody or polypeptide via sulfhydryl group.

In one embodiment, a targeting ligand may comprise a succinimide group, wherein the succinimide group is attached to an antibody or polypeptide via amino group.

In one embodiment, a targeting ligand can be a targeting polypeptide molecule, such as prostate-specific membrane antigen (PSMA), arginine (R)-glycine (G) aspartic acid (D) tripeptide, and chemokine receptor (CXCR4), etc.

In one embodiment, a targeting ligand is a lateral ligand or an axial ligand. In a preferred embodiment, the targeting ligand is an axial ligand.

In some embodiments, the metal complex of general formula (I) is a tetravalent platinum complex as shown below, 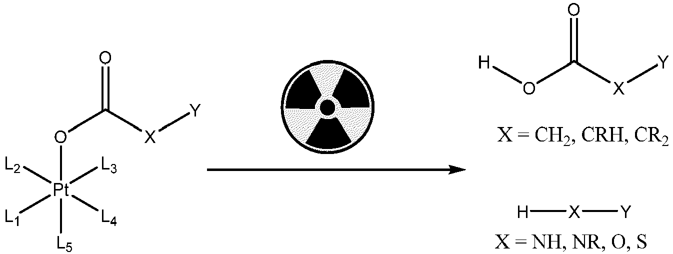 wherein H-OC(O)-X-Y or H-X-Y is a functional molecule.

Axial ligand L₅ is also a leaving group, which can be divided into three categories:
**1.** a non-functional capping agent, such as acetic acid and other molecules that do not have therapeutic or targeting effects;
**2.** a targeting molecule, which can be maleimide-containing molecules for linking with antibody or polypeptide via sulfhydryl groups; or succinimide-containing molecules for linking with antibody or polypeptide via amino groups; or directly linked polypeptide molecules with targeting effect, such as PSMA, RGD, CXCR4, etc.;
**3.** a therapeutic molecule that is the same as another axial ligand, or different from another axial ligand, wherein two drugs may play a synergism role.

In one embodiment, the ligand P in the complex is obtained by the reaction shown below.

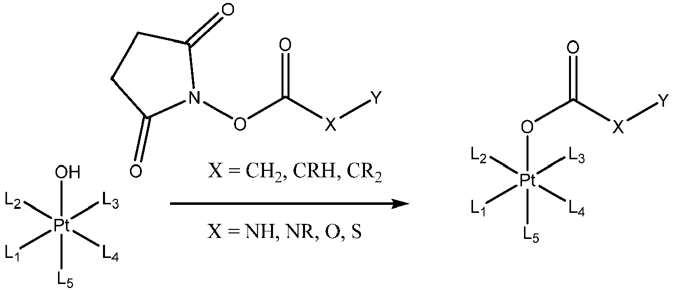

Hydroxylated tetravalent platinum and an equivalent molar amount of active ester are stirred in dimethyl sulfoxide at ambient temperature, such as room temperature, for example for 24 hours, to obtain the target product. The solvent is lyophilized; and then the solid is washed with organic solvents such as diethyl ether and ethanol in sequence to obtain the purified target product. Active esters are either commercially available or prepared by conventional synthetic methods of organic chemistry. Active esters can be produced, for example, by the following method. 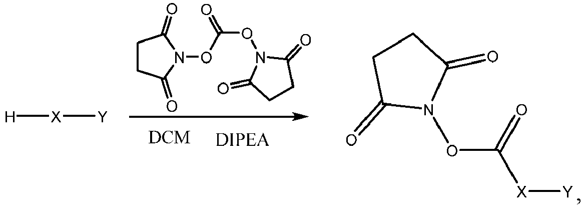 wherein X is NH, NR, O or S.

Y-XH is dissolved in dichloromethane. An equivalent molar amount of disuccinimidyl carbonate is added, followed by an equivalent molar amount of diisopropylethylamine (DIPEA). The mixture is reacted for 12 hours, and then purified by silica gel column to obtain the active ester. 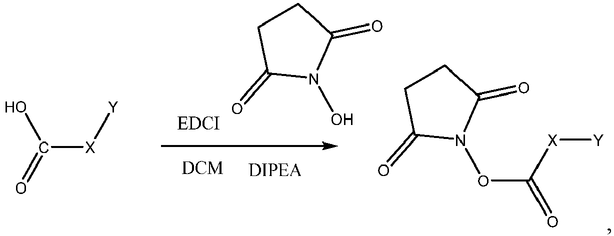 wherein X is CH₂, CRH or CR₂.

A carboxylic acid Y-X-COOH is dissolved in dichloromethane. Two equivalents of diisopropylethylamine are added. One equivalent of condensing agent 1-ethyl-(3-dimethylaminopropyl)carbodiimide hydrochloride (EDCI), and then N-hydroxysuccinimide is added. The mixture is reacted for 12 hours, and then purified by silica gel column to obtain the active ester.

In one embodiment, the present disclosure uses a primary or secondary amine-containing drug as a functional molecule. In a preferred embodiment, an anticancer drug containing a primary or secondary amine is used as a functional molecule. Drugs containing a primary or secondary amine include, for example, ibrutinib, acatinib, zanubrutinib, doxorubicin, mitomycin-C, mitomycin-A, daunorubicin, aminopterin, actinomycin, bleomycin, 9-aminocamptothecin, N8-acetylspermidine, 1,2-bis(methylsulfonyl)-1-(2-chloroethyl)hydrazine, yunnanmycin, gemcitabine, cytarabine, dolastatin, dacarbazine, 5-fluorouracil, and their derivatives. Drugs containing a primary or secondary amine also include amino derivatives of drugs that do not naturally contain amino groups. In other words, drugs that do not originally contain amino groups can be chemically modified to have amino groups, and then active esters are prepared through primary or secondary amines and further reacted with hydroxylated tetravalent platinum to obtain tetravalent platinum coordinated with ⁻O(O)C-X-Y ligand.

In a preferred embodiment, the functional molecule is monomethyl auristatin E.

In one embodiment, the present disclosure uses a hydroxyl-containing drug as a functional molecule. In a preferred embodiment, a hydroxyl-containing anticancer drug is used as a functional molecule. Hydroxyl-containing functional molecules include, for example, paclitaxel, docetaxel, gemcitabine, cytarabine, and the like. Hydroxyl-containing functional molecules also include hydroxy derivatives of drugs that do not naturally contain hydroxyl groups. In other words, a drug that does not contain a hydroxyl group can be chemically modified to have a hydroxyl group, and then an active ester is prepared through the hydroxyl group and further reacted with hydroxylated tetravalent platinum to obtain tetravalent platinum coordinated with ⁻O(O)C-X-Y ligand.

In one embodiment, the present disclosure uses a sulfhydryl-containing drug as a functional molecule. In a preferred embodiment, a sulfhydryl-containing anticancer drug is used as a functional molecule. The functional molecules containing a sulfhydryl group include, for example, 6-mercaptopurine and the like. The sulfhydryl-containing functional molecules also include sulfhydryl derivatives of drugs that do not naturally contain sulfhydryl groups. In other words, drugs that do not originally contain sulfhydryl groups can be chemically modified to have a sulfhydryl group, and then active esters can be prepared through sulfhydryl groups and further reacted with hydroxylated tetravalent platinum to obtain tetravalent platinum coordinated with ⁻O(O)C-X-Y ligand.

Similar to drug molecules, active esters can also be prepared by other functional molecules, and further be reacted with hydroxylated tetravalent platinum to obtain tetravalent platinum coordinated with ⁻O(O)C-X-Y ligand.

### Example

The starting materials for the examples are commercially available and/or can be prepared in a variety of ways well known to those skilled in the art of organic synthesis. Those skilled in the art of organic synthesis will appropriately select reaction conditions (including solvent, reaction atmosphere, reaction temperature, duration of experiment, and workup) in the synthetic methods described below. Those skilled in the art of organic synthesis will understand that the functional groups present on each part of the molecule should be compatible with the proposed reagents and reactions. NMR was recorded using a Bruker AVANCE 400 MHz spectrometer. High-resolution mass spectra were measured using a Bruker Fourier Transform Ion Cyclotron resonance mass spectrometer. The liquid chromatography-mass spectrometry used was a Waters e2695 instrument equipped with a Waters 2995 PDA and a Waters Acquity QDA mass spectrometer.

The change of the valence state of metal ions will involve the transfer of electrons. First, a large number of metal ions and metal complexes have been studied on chemical reactions driven by high-energy rays.

The following metal salts and metal complexes were screened:

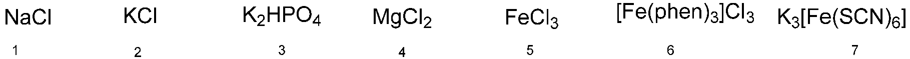

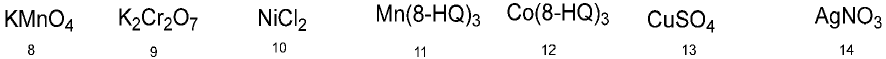

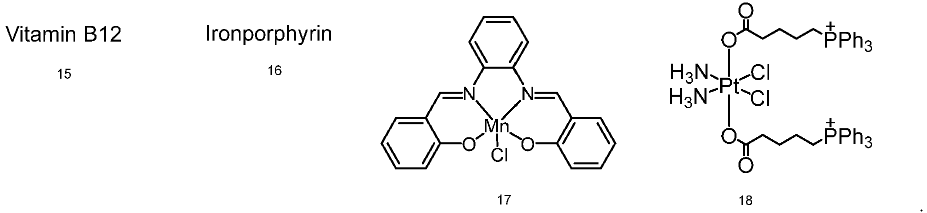

Operation steps of test: the compound was dissolved in ultrapure water to prepare a 100 µM metal compound solution, and oxygen gas in the solution was removed by bubbling nitrogen gas for 15 minutes. The deoxidized metal solution was irradiated with X-rays from radiotherapy apparatus (RAD-SOURCE, model RS 2000-225, irradiation parameter: 4Gy/min) to receive X-rays at a dose of 0-1000 Gy. Finally, the remaining amount of raw material was measured, the disappeared concentration of the reactant was calculated, and the radiation yield was calculated (radiation yield = disappeared concentration of reactant / radiation dose).

Table 1. Yield of reduced metal compounds under irradiation

| Sample code and sample name | Yield (nM/Gy) |
|---|---|
| 1 (sodium chloride) | - (No metal cation reduction reaction occured) |
| 2 (potassium chloride) | - (No metal cation reduction reaction occured) |
| 3 (dipotassium hydrogen phosphate) | - (No metal cation reduction reaction occured) |
| 4 (magnesium chloride) | - (No metal cation reduction reaction occured) |
| 5 (ferric chloride) | 578 |
| 6 | 566 |
| 7 | 572 |
| 8 (potassium permanganate) | 64 |
| 9 (potassium dichromate) | 87 |
| 10 (nickel chloride) | 347 |
| 11 | 655 |
| 12 | 478 |
| 13 (copper sulfate) | 209 |
| 14 (silver nitrate) | 576 |
| 15 (vitamin B12) | 553 |
| 16 (iron porphyrin) | 598 |
| 17 | 575 |
| 18 | 427 |

Summarizing the above results, it was found that compounds with two or more stable valence states in aqueous solution can be reduced. At the same time, it was found in tests that for metal complexes, accompanied by the reduction of metal ions, the complexes usually had structural changes, and even released ligand compounds.

### Molecular synthesis route:

Molecules 1-17 were all commercially available compounds.

### General synthetic route for molecules 19-28:

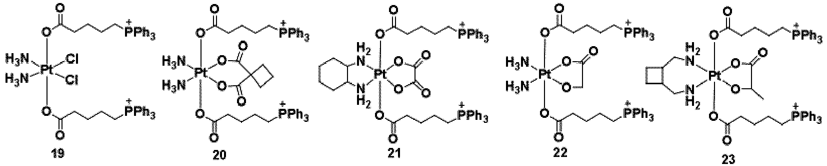

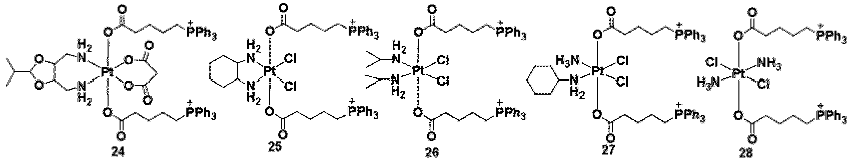

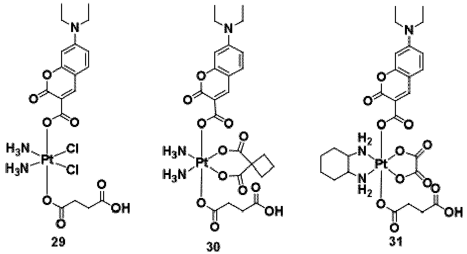

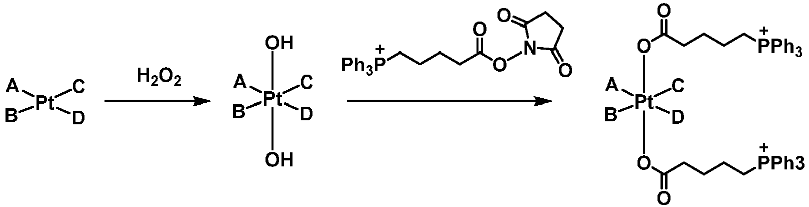

Dihydroxylated tetravalent platinum was obtained by reacting commercially available divalent platinum (1 mmol) with 3 mL of hydrogen peroxide for 3 hours, and then filtering. The target compound was obtained by reacting tetravalent platinum with the active ester of triphenylphosphonium (2.2 mmol).
Compound 19 detected by mass spectrometry: 511.62 (with two positive charges)
Compound 20 detected by mass spectrometry: 547.66 (with two positive charges)
Compound 21 detected by mass spectrometry: 560.67 (with two positive charges)
Compound 22 detected by mass spectrometry: 513.65 (with two positive charges)
Compound 23 detected by mass spectrometry: 560.69 (with two positive charges)
Compound 24 detected by mass spectrometry: 597.69 (with two positive charges)
Compound 25 detected by mass spectrometry: 551.65 (with two positive charges)
Compound 26 detected by mass spectrometry: 553.67 (with two positive charges)
Compound 27 detected by mass spectrometry: 553.16 (with two positive charges)
Compound 28 detected by mass spectrometry: 511.62 (with two positive charges)

### General synthetic route for molecules 29-31:

The target products 29-31 were obtained by reacting hydroxylated tetravalent platinum with an equivalent molar amount of active ester and then adding two equivalents of succinic anhydride. 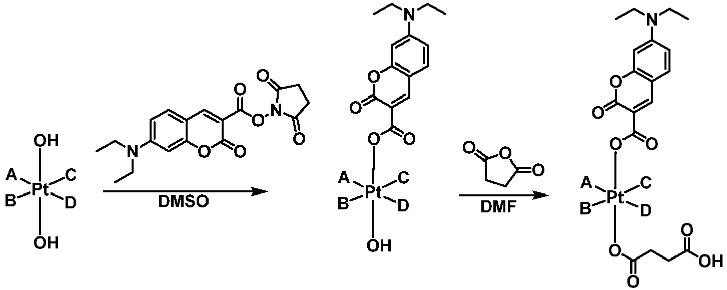
Compound 29 detected by mass spectrometry: 675.06 (with one negative charge)
Compound 30 detected by mass spectrometry: 747.16 (with one negative charge)
Compound 31 detected by mass spectrometry: 773.17 (with one negative charge)

### General synthetic route for molecules 32-34:

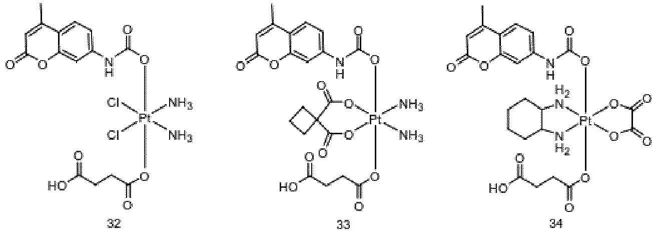

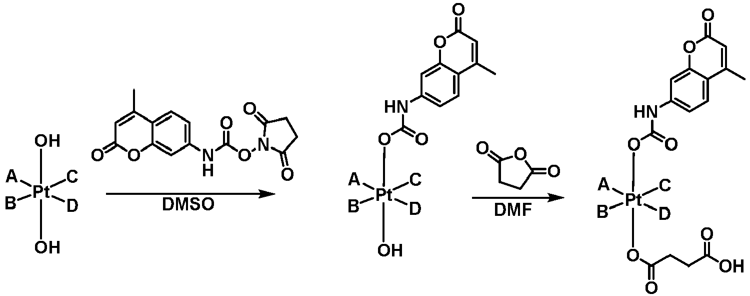

The target products 32-34 were obtained by reacting hydroxylated tetravalent platinum with an equivalent molar amount of active carbamate and then adding two equivalents of succinic anhydride.
Compound 32 detected by mass spectrometry: 633.01 (with one negative charge)
Compound 33 detected by mass spectrometry: 705.10 (with one negative charge)
Compound 34 detected by mass spectrometry: 731.11 (with one negative charge)

### Synthetic steps of molecule 35

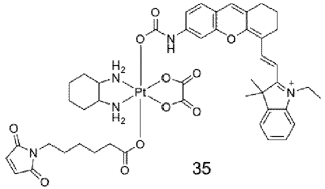

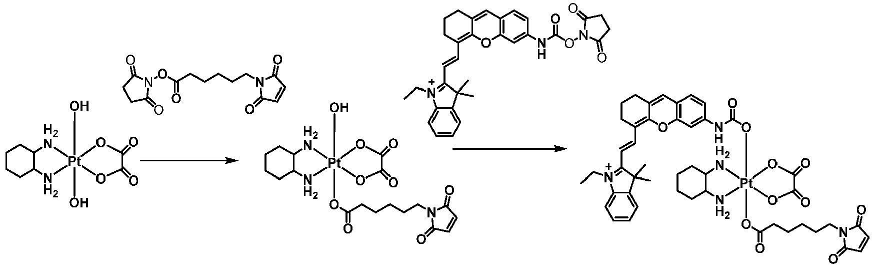

Tetravalent platinum was obtained by oxidizing oxaliplatin, and then reacted with an equivalent molar amount of active ester, and further reacted with an equivalent molar amount of active carbamate to obtain product 35.
Molecule 35 detected by mass spectrometry: 1047.35 (with one positive charge)

### Synthesis of molecules 36-38

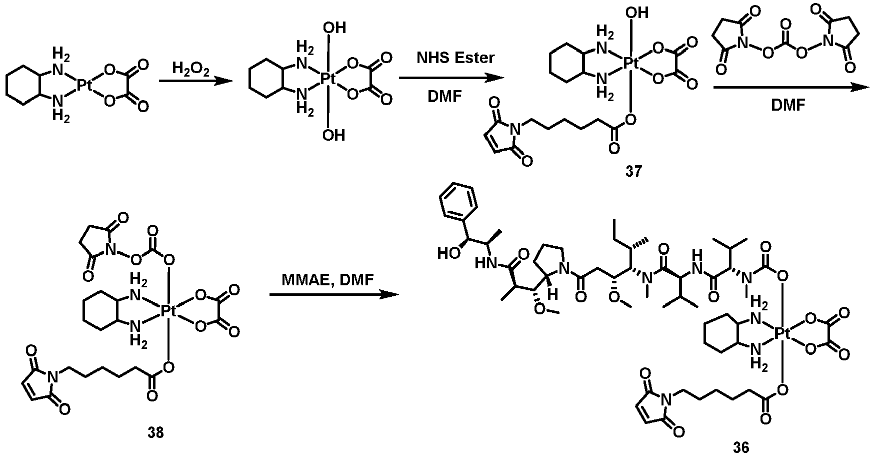

Dihydroxylated tetravalent platinum was obtained by reacting commercially available oxaliplatin (10 mmol) with 30 mL of hydrogen peroxide for 3 hours and then filtering.

Compound 37 was obtained by reacting tetravalent platinum with an active ester of maleimide (12 mmol) in 10 mL of DMF for 12 hours, and then purified by silica gel column chromatography with DCM:MeOH=9:1 as an eluent.

Compound 38 was obtained by dissolving compound 37 in DMF, adding succinimidyl carbonate (24 mmol), and reacting for 6 hours, and then purified by silica gel column chromatography with DCM:MeOH=20:1 as an eluent.

Compound 36 was obtained by dissolving compound 38 in DMF, adding MMAE (10 mmol), and reacting for 24 hours, and then purified by silica gel column chromatography with DCM:MeOH=20:1 as an eluent.
Molecule 37 detected by mass spectrometry: 625.14 (positive ion peak with one hydrogen added)
Molecule 38 detected by mass spectrometry: 766.15 (positive ion peak with one hydrogen added)
Molecule 36 detected by mass spectrometry: 1368.62 (positive ion peak with one hydrogen added)

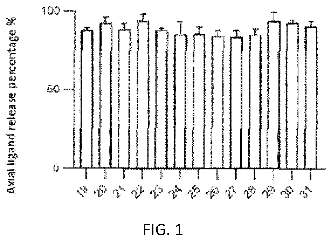

We performed studies with platinum-based complexes to determine the feasibility of this radiation-activation approach in vivo. Firstly, the tetravalent platinum complex was irradiated with high-energy rays to prove that the reaction has a broad spectrum for platinum-based drugs, and the results are shown in Fig. 1.

Operation steps of test: the tetravalent platinum complex was dissolved in ultrapure water to prepare a 10 µM solution, and at the same time oxygen gas in the solution was removed by bubbling nitrogen gas. The deoxygenated tetravalent platinum complex solution was irradiated by X-rays from radiotherapy apparatus (RAD•SOURCE, model RS 2000-225, irradiation parameter: 4Gy/min) to receive X-rays at a dose of 0-60 Gy. The peak area of the newly generated axial ligand peak was detected by ultra-high performance liquid chromatography, and the release of axial ligand during irradiation was determined by the standard curve of the peak area-concentration plot of the pure ligand in HPLC.

The test results of the ligand release ratio (actual release of axial ligand after reaction to theoretically complete release of axial ligand) of 10 µM tetravalent platinum complexes 19-31 after 60 Gy of X-ray irradiation were shown in Fig. 1. After the detection of radiation activation of molecules 19 to 31, it was confirmed that the reduction of tetravalent platinum metal complexes by high-energy rays and the release of axial ligands have broad spectrum.

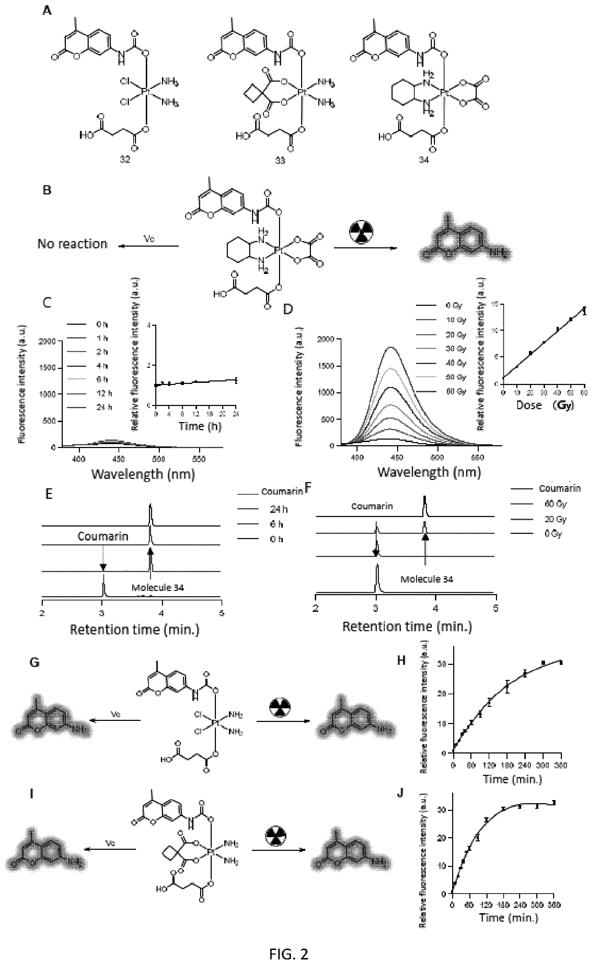

To apply this radiation-activated chemical reaction in vivo, a problem must be solved, i.e., the complex needs to keep stable in the reducing environment in vivo. Platinum drugs that have been approved by the FDA include cisplatin, carboplatin and oxaliplatin. In the tetravalent platinum precursors that use these three platinum drugs as the parent, is there any tetravalent platinum drug that is stable in the reducing environment in vivo, especially in tumors? At the same time, does this method only release carboxyl ligands? Compounds 32, 33 and 34 were used for stability screening because there were a large number of drugs containing amino groups. The result was shown in Fig. 2.

Steps of test: Compounds 32, 33, and 34 were respectively dissolved in pure water to prepare a 10 µM solution, and at the same time, nitrogen gas was bubbled to remove oxygen gas in the solution. The deoxygenated tetravalent platinum complex solution was irradiated by X-rays from radiotherapy apparatus (RAD•SOURCE, model RS 2000-225, irradiation parameter: 4Gy/min) to receive X-rays at a dose of 0-60 Gy. The release of axial ligands was detected by ultra-high performance liquid chromatography. The results of the compound's response to high-energy radiation were thus obtained.

32, 33, and 34 were dissolved respectively in pure water to prepare a 10 µM solution, and at the same time oxygen gas in the solution was removed. Endogenous reducing substance vitamin C was then added, so that the concentration of vitamin C was 2 mM. The co-incubation time was 24 h, and the release of axial ligands in different time periods was detected. The stability of the compounds under reducing environments was thus determined.

A tetravalent platinum complex with oxaliplatin as the parent was formulated to obtain a 10 µM solution, and oxygen gas was removed from the solution. Various types of endogenous reducing substances (cysteine, glutathione, reduced nicotinamide adenine dinucleotide phosphate) were then added at different concentrations. In this way, the stability of the compounds against endogenous reducing substances in the cellular environment or in vivo environment was determined.

Fig. 2 is the test results of screening tetravalent platinum compounds 32, 33 and 34 on stability in vivo. In Fig. 2, (A) shows the structures of the three platinum compounds used in the study; (B), (G), and (I) are the reaction schematic diagrams of the three tetravalent platinum compounds; (C) is the fluorescence change diagram of molecule 34 after co-incubation with Vc, and it can be seen that molecule 34 was relatively stable in the presence of Vc, and no reduction reaction occurred; (D) is the fluorescence change diagram of molecule 34 after being irradiated, and it can be seen that the relative fluorescence intensity is linearly correlated with the irradiation dose; (E) is the UPLC change diagram after co-incubation of molecule 34 and Vc, and it can be seen that no new coumarin peak was generated after co-incubation of molecule 34 and Vc; (F) is the UPLC change diagram of molecule 34 after receiving irradiation, and it can be seen that during the irradiation, the axial ligand coumarin was gradually released with the increase of irradiation dose; (H) is the fluorescence change diagram of molecule 32 after co-incubation with Vc; (J) is the fluorescence change diagram of molecule 33 after co-incubation with Vc.

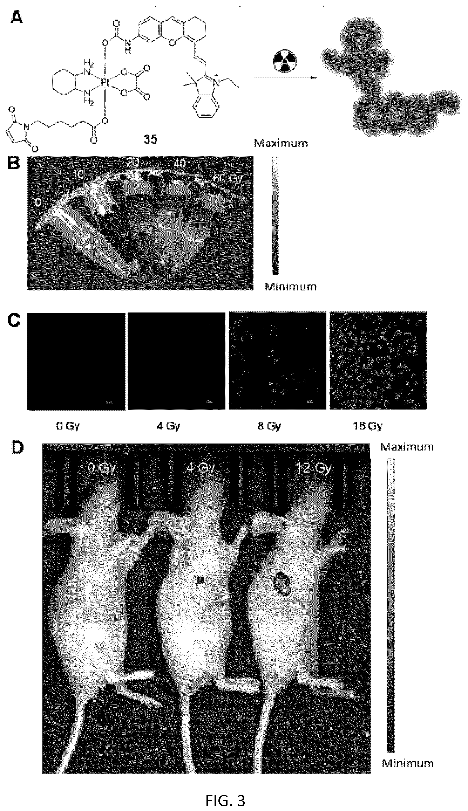

After confirming that the compound with oxaliplatin as the parent was stable and can be activated by high-energy rays in a test tube, the feasibility of this activation method in vivo was studied. Molecule 35 was designed so that ligand released upon activation can emit the near-infrared fluorescence, and the axial ligand release in vivo can be nondestructively determined. The test result is shown in Fig. 3.

### Test steps:

Test in test tube: molecule 35 was dissolved in pure water to prepare a 10 µM solution, and at the same time oxygen gas in the solution was removed. The deoxygenated tetravalent platinum complex solution was irradiated by X-rays from radiotherapy apparatus (RAD•SOURCE, model RS 2000-225, irradiation parameter: 4Gy/min) to receive X-rays at a dose of 0-60 Gy. Change in the fluorescence signal of the solution was then detected with a small animal imager.

Cell test: molecule 35 was dissolved in HBSS buffer to prepare a 10 µM solution, and at the same time oxygen gas in the solution was removed. Then the solution was incubated with the cells, and then the mixture was irradiated with X-rays from the radiotherapy apparatus to receive X-rays at a dose of 0-16 Gy. The fluorescent signal of the cells was detected with a confocal microscope.

In vivo test: molecule 35 was dissolved in DMSO to prepare a 20 mM stock solution. 20 µL of phosphate buffer solution (pH = 7.4) containing 1% DMSO (in which the concentration of 35 was 200 µM) was injected into the tumor area of mice. Tumors were then irradiated locally with X-rays at doses of 0, 4, and 12 Gy, respectively. The fluorescence signal of the tumor area was then detected with a small animal imager.

Fig. 3 shows an in vivo activation test of tetravalent platinum with oxaliplatin as the parent. In Fig. 3, (A) is schematic diagram of the structure and activation of molecule 35; (B) is schematic diagram of the release of axial ligands by molecule 35 in a test tube; (C) is a confocal image of molecule 35 activated in a cell environment; (D) is a small animal imaging image of molecule 35 activated in a mouse tumor model.

Test results showed that molecule 35 can be reduced to release ligands by high-energy rays in test tubes, cells and mouse models in vivo, and this release strategy has been proved in concept.

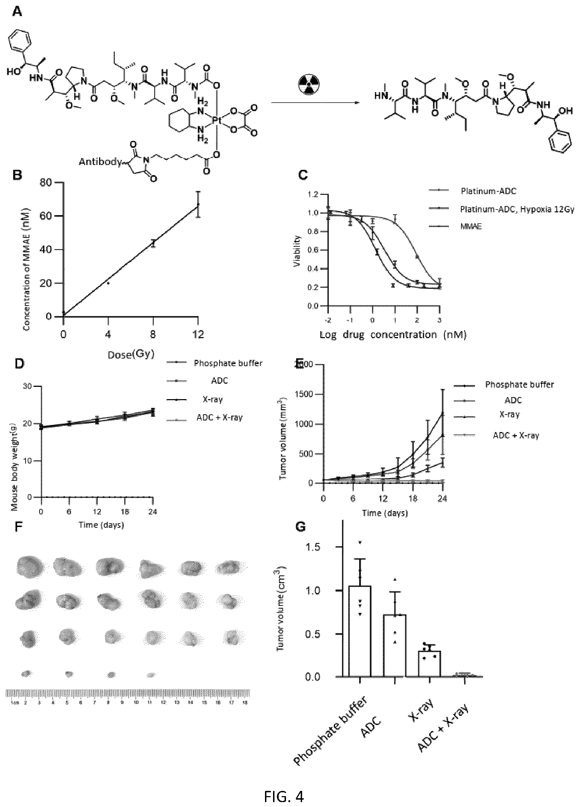

Activation of chemotherapeutics by radiotherapy is in line with the goal of precision medicine, which can be achieved with antibody-drug conjugates. The prodrug compound 36 was prepared by using the oxaliplatin parent as a linker, and the antibody-drug conjugate was prepared by linking 36 with the antibody Herceptin. The antibody-drug conjugate was studied in a mouse treatment test, and the result was shown in Fig. 4.

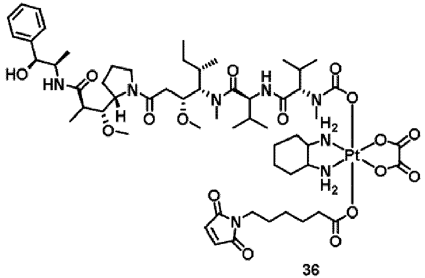

### Test steps:

Preparation of antibody-drug conjugate (ADC): 200 µL of phosphate buffered saline solution was added to a 1.5 mL centrifuge tube, then 100 µL of 50 mg/mL Herceptin solution was added. Afterwards, 75 µL of 1 mM TECP was added and reacted for 1.5 h for opening disulfide bond. Then, 35 µL of 10 mM compound 36 in DMSO was added to the reaction solution, and reacted for 1 h. Unreacted small molecules were removed by ultrafiltration centrifugation to obtain antibody-drug conjugate.

Antibody-drug conjugate release test in vitro: the prepared antibody-drug conjugate was diluted to 50 nM, and irradiated with 0-16 Gy of X-ray (RAD•SOURCE, model RS 2000-225, irradiation parameter: 4 Gy/min). The mass spectrum signal intensity of the functional molecule MMAE was detected by mass spectrum. The release amount of MMAE was determined through the external standard curve, that is, the MMAE concentration-mass spectrum signal intensity curve.

Cytotoxicity test: different concentrations of antibody-drug conjugates were incubated with cells and irradiated with different doses of X-rays to determine the half-inhibitory concentration of antibody-drug conjugates on cancer cells under different conditions.

Animal treatment test: the mice were divided into four groups, wherein the first group was only injected with PBS solution, the second group was injected with PBS and then irradiated with X-ray, the third group was only injected with platinum antibody-drug conjugate, and the fourth group was injected with the antibody-drug conjugate and then irradiated with X-ray. Tumor volume was measured.

Fig. 4 is a radiation-responsive antibody-drug conjugate with platinum as a linker. In Fig. 4, (A) shows schematic diagram of the activation of antibody-drug conjugate (ADC) to release MMAE; (B) shows release test of antibody-drug conjugate (50 nM) in a test tube; (C) shows cytotoxicity test of antibody-drug conjugate; (D) shows body weight change curve of mice; (E) shows mice tumor growth curve; (F) shows tumor image on day 24; (G) shows tumor mass image on day 24.

This treatment method can significantly inhibit the growth of tumors, and has extremely high clinical application value.

The examples of the present disclosure demonstrate in principle the feasibility of a strategy of irradiating tetravalent platinum complexes to release functional molecules. Further, the examples of the present disclosure use a MMAE functional molecule as a model to illustrate the feasibility of the strategy of irradiating tetravalent platinum complexes to release drug molecules. The examples of the present disclosure also demonstrate that the concentration of a functional molecule released from a complex has a good linear relationship with a dose.

A prodrug is an inactive substance that is converted into an active drug in the body by the action of enzymes or other chemicals. This approach is widely used in the pharmaceutical industry and requires the release of sufficiently high concentrations of the drug at the desired location. Antibody-drug conjugate (ADC) is a perfect example, where its tumor selectivity makes it promising, but is often hampered by difficult-to-control drug release. In fact, classical ADC (such as TDM-1) requires receptor-mediated endocytosis for drug release, and the therapeutic effect on heterogeneous tumors is not satisfactory. To improve therapeutic efficacy and reduce side effects, scientists have developed many drug release strategies. In clinical research, ADC linkers are often non-cleavable, or require endogenous stimuli such as acidic conditions, lysosomal proteases, and GSH to initiate cleavage. Few release strategies mediated by exogenous stimuli (such as small chemical molecules) have entered clinical research, mainly because side effects caused by small chemical molecules are difficult to be assessed in clinical trials. Therefore, we attempted to investigate whether radiation-driven cleavage chemistry could be applied to the release of ADC-loaded drugs to improve the release efficiency of drugs in tumors. Firstly, using an oxaliPt(IV)-OH carbamate linker, monomethyl auristatin E (MMAE) was linked to monoclonal anti-HER2 antibody trastuzumab, a commonly used ADC system (Fig. 6a). The OxaliPt(IV)-carbamate linker responds selectively to X-ray-generated e_{aq}⁻ and further decarboxylates to release an axial ligand, giving highly toxic free MMAE (Fig. 6a).

Following the established method, 2.2 equivalents of TECP were used to reduce trastuzumab to free thiols without reducing the oxaliPt(IV) complex, and then 10 equivalents of MMAE were directly added to the reaction. The crude product was purified by PD-10 column. High performance liquid chromatography (HPLC) showed that the trastuzumab-oxaliPt(IV)-MMAE (oxaliPt(IV)-ADC) conjugate had a main peak (Fig. 6b); MALDI-TOF-MS analysis showed that the main peak of each antibody contained 4 payloads (Fig. 6c), which was the optimal drug-to-antibody ratio (DAR) for tumor-selective drug delivery in vivo.

To test whether radiation-driven release would achieve drug release from ADCs, we chosed to use BGC823 cells, a human gastric cancer cell line with moderate levels of HER2 expression, for cell viability tests of oxaliPt(IV)-ADC+X-rays (Fig. 6d). BGC823 cells were treated with only oxaliPt(IV)-ADC under hypoxic conditions, with only oxaliPt(IV)-ADC under normoxia conditions, and with oxaliPt(IV)-ADC+8 Gy X-rays under normoxia conditions. After 96 hours of culture, CCK8 detection showed that the cell viability in the oxaliPt(IV)-ADC+8 Gy X-rays treatment group was significantly decreased, with IC50 of 2.60±1.60 nM. In contrast, the IC50 of oxaliPt(IV)-ADC was 85.90±24.16 nM. Therefore, radiation-driven oxaliPt(IV)-ADC was 33 times more cytotoxic than oxaliPt(IV)-ADC in a BGC823 cell line. Under hypoxic conditions, oxaliPt(IV)-ADC showed no observable cytotoxicity, indicating good in vitro stability of oxaliPt(IV)-ADC.

Fig. 6 depicts the construction and characterization of ADCs whose drug release can be driven by radiotherapy: **a,** schematic diagram of the structure of trastuzumab-Pt(IV)-MMAE conjugated drug (oxaliPt(IV)-ADC), and the X-ray-driven release of the drug MMAE thereof. **b**, the purity of oxaliPt(IV)-ADC determined by HIC-HPLC. **c,** MALDI-TOF analysis showed that the drug-antibody ratio of oxaliPt(IV)-ADC was about 4. **d,** Cell survival rate of BGC823 cells treated with MMAE, oxaliPt(IV)-ADC, oxaliPt(IV)-ADC+8 Gy X-rays.

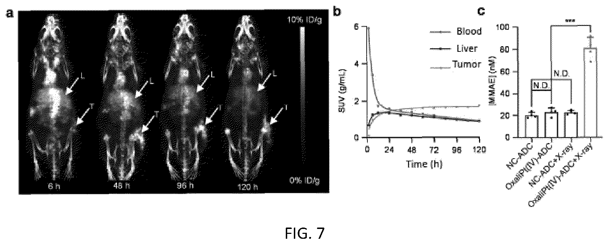

When using Positron Emission Tomography-Computed Tomography (PET-CT) imaging to study the pharmacokinetics of oxaliPt(IV)-ADC in BGC823 tumor-bearing mice, firstly, oxaliPt(IV)-ADC was labeled with ⁸⁹Zr-Zr(oxalate)₂. PET-CT imaging at multiple time points **(****Fig. 7a****)** showed that ⁸⁹Zr-oxaliPt(IV)-ADC showed good blood circulation, and tumor uptake began 6 hours after administration, and gradually increased with time, until reaching equilibrium at 36 hours (10% ID/g, **Fig. 7b****)**. After 48 hours of administration, the radioactive uptake in the tumor was significantly higher than that in the blood, and the drug content in the blood continued to decrease, while the drug uptake of the tumor continued to increase **(****Fig. 7b****).** Studying the pharmacokinetics of drugs helps to understand the dynamic distribution of ADCs in mice and determine the correct time for tumor irradiation to obtain the optimal time point of radiotherapy and chemotherapy. As shown in Fig. 7d, BGC823 tumor mice were randomly divided into 4 groups: control groups (non-cleavable ADC (NC-ADC), oxaliPt(IV)-ADC, NC-ADC+X-ray) and oxaliPt(IV)-ADC+X-ray treatment group. All mice were injected with 5 mg/kg ADC on day 0. For the treatment group, 8 Gy of X-rays were given on day 2 after ADC injection according to the previous in vivo biodistribution. The mice were then dissected, and xenogeneic tumors were collected. The intratumoral concentration of MMAE was analyzed by LC-QTOF-MS. The results showed that the drug release of MMAE in the treatment group was at least 3 times higher than that in the group using oxaliPt(IV)-ADC alone. For NC-ADC, there was no difference in the release of MMAE with or without X-rays, indicating that the release of MMAE depends on the reduction of Pt(IV) by X-rays. It indicates the feasibility of radiation-driven chemical cleavage reaction to release drugs and realize tumor therapy.

Fig. 7 depicts the pharmacokinetic study of OxaliPt(IV)-ADC: a, PET-CT images of BGC823 tumor-bearing mice after intravenous injection of [⁸⁹Zr]oxaliPt(IV)-ADC. **b,** Time-activity curves (TAC) of [⁸⁹Zr]oxaliPt(IV)-ADC in blood, liver and tumor. **c**, MMAE concentrations in tumors detected after mice were treated with oxaliPt(IV)-ADC (5 mg/kg) or NC-ADC (5 mg/kg) alone, or with oxaliPt(IV)-ADC (5 mg/kg) + X-rays (8 Gy), or with NC-ADC (5 mg/kg) + X-rays (8 Gy).

The above descriptions are only exemplary embodiments of the present disclosure, and are not intended to limit the protection scope of the present disclosure, which is determined by the appended claims.

This application claims the priority of the Chinese patent application No. 202011337782.X filed on November 25, 2020, and the content disclosed in the above Chinese patent application is incorporated in its entirety as a part of this application.

## Claims

1. A metal complex of general formula (I),
Lₓ-M-P (I)
wherein:
M is tetravalent platinum;
L is independently for each occurrence a neutral ligand or an anionic ligand;
x is an integer from 1 to 5; and
P is a precursor ligand, the precursor ligand being a ligand of the tetravalent platinum ion which can be released from the complex after irradiation and converted into a functional molecule D.

2. The metal complex according to claim 1, wherein the functional molecule D is selected from drug molecules, fluorescent molecules, and functional material molecules.

3. The metal complex according to claim 2, wherein the functional molecule D is an anticancer drug molecule.

4. The metal complex according to claim 3, wherein at least one L has a group targeting tumor cells.

5. The metal complex according to claim 4, wherein the L having a group targeting tumor cells includes a sugar transporter targeting group, a glutamine receptor targeting group, a phosphate receptor targeting group, an epidermal growth factor receptor targeting group, an integrin targeting group, an energy metabolism enzyme targeting group, a chondriosome targeting group, a serum albumin targeting group, an inflammatory factor targeting group, a DNA targeting group, a histone deacetylase (HDAC) targeting group, a P53 gene activator group, a tubulin inhibitor group, a cyclin-dependent kinase inhibitor group, or an indoleamine 2,3-dioxygenase inhibitor group.

6. The metal complex according to any one of claims 2-5, wherein the functional molecule D is selected from monomethyl auristatin E, monomethyl auristatin F, ibrutinib, acatinib, zanubrutinib, doxorubicin, mitomycin-C, mitomycin-A, daunorubicin, aminopterin, actinomycin, bleomycin, 9-aminocamptothecin, N8-acetylspermidine, 1,2-bis(methylsulfonyl)-1-(2-chloroethyl)hydrazine, yunnanmycin, gemcitabine, cytarabine, dolastatin, dacarbazine, 5-fluorouracil, paclitaxel, docetaxel, gemcitabine, cytarabine, and 6-mercaptopurine.

7. The metal complex according to claim 6, wherein the functional molecule D is monomethyl auristatin E, monomethyl auristatin F, or 5-fluorouracil.

8. The metal complex according to any one of claims 1-7, wherein at least one ligand L is selected from: NH₃, ethylenediamine, F⁻, Cl⁻, oxalate, malonate, 1,2-diaminocyclohexane, 1,2-diaminobenzene, 2-aminopropane, aminocyclohexane, cyclobutane-1,1-dicarboxylate, glycolate, lactate, aminocyclohexane, 2-isopropyl-4,5-bis(aminomethyl)-1,3-dioxolane, 5-(triphenylphosphonio)valerate, succinate, 6-(2,5-dioxo-2,5-dihydro-1H-pyrrol-1-yl)hexanoate, porphyrin, acetate, and propionate.

9. The metal complex according to any one of claims 1-8, wherein one ligand L is an axial ligand capable of being released from the complex after irradiation and converted into a functional molecule which may be the same as or different from the functional molecule D.

10. The metal complex according to any one of claims 1-9, wherein P is an axial ligand.

11. The metal complex according to any one of claims 1-10, wherein P is ⁻O-C(=O)-X-Y, wherein
X is -NH-, -NR-, -O-, or -S-, R is an optionally substituted C₁₋₁₀ alkyl, and HXY constitutes the functional molecule D; or
X is -CH₂-, -CRH-, or -CR₂-, wherein R is independently for each occurrence an optionally substituted C₁₋₁₀ alkyl, or two R groups are taken together with the carbon atom to which they are attached to form a 5-membered ring or a 6-membered ring, and HOOCXY constitutes the functional molecule D.

12. The metal complex according to any one of claims 1-11, wherein the metal complex of general formula (I) can release an axial ligand from the complex to obtain a divalent platinum complex after irradiation.

13. The metal complex according to claim 12, wherein the divalent platinum complex has an anticancer effect.
